# FASCICULE DE BREVET EUROPEEN

(11) **EP 1 924 218 B1**
(45) Date de publication et mention de la délivrance du brevet: **11.04.2012**
(21) Numéro de dépôt: 06808124.9
(22) Date de dépôt: 13.09.2006
(51) Int. Cl.: A61F 2/00

(54) **DISPOSITIF DE PRÉVENTION DE L'INCONTINENCE URINAIRE FÉMININE À L 'EFFORT**
VORRICHTUNG ZUM VERHINDERN VON STRESSBEDINGTER INKONTINENZ BEI DER FRAU
DEVICE FOR PREVENTING FEMALE STRESS INCONTINENCE

(30) Priorité: 14.09.2005 FR 0509383
(43) Date de publication de la demande: 28.05.2008
(73) Titulaire: B.Braun Medical SAS, 92100 Boulogne-Billancourt (FR); Guerquin, Bernard, 57000 Saint-Avold (FR)
(72) Inventeur: GUERQUIN, Bernard, F-57000 Saint-Avold (FR); COLLIN, Rémi, F-61340 Saint Hilaire sur Erre (FR)
(74) Mandataire: Lebrette, Camille
(86) Numéro de dépôt international: PCT/FR2006/002098
(87) Numéro de publication internationale: WO 2007/031645

(56) Documents cités:
- EP-A- 0 264 258
- WO-A-00/36996
- AU-B2- 614 940
- US-A- 3 705 575
- US-A- 5 386 836
- US-A1- 2002 083 949
- US-B1- 6 679 831

## Description

La présente invention concerne un dispositif de prévention de l'incontinence urinaire féminine à l'effort, destiné à être disposé longitudinalement dans un vagin.

On connaît déjà des dispositifs de prévention de l'incontinence urinaire féminine à l'effort. Par exemple, le document US-5 785 640 décrit un dispositif introduit profondément dans la zone sous-vésicale du vagin de manière que son extrémité antérieure exerce une pression à l'emplacement du col vésical. La partie sous-urétrale du vagin reste pratiquement libre.

Le document US-5 386 836 décrit un dispositif intra-vaginal comportant un anneau qui porte une ou deux saillies dépassant de l'anneau dans une direction faisant un angle de 90 à 135° avec le plan de l'anneau ; les saillies sont destinées à soutenir le col vésical. En conséquence, les saillies appliquent une pression de part et d'autre du col vésical ou sur le col vésical, mais elles ne pénètrent pas dans la zone sous-urétrale, puisqu'elles se limitent au col vésical.

Le document US-6 679 831 décrit un dispositif de prévention de l'incontinence urinaire féminine à l'effort qui comprend un organe élastique interne, et un matériau non absorbant. L'organe élastique est destiné à donner au dispositif, après qu'il a été extrait d'un applicateur, une forme qui s'élargit au niveau du col vésical qui est ainsi soumis à une pression locale. Ce document décrit des dispositifs pouvant prendre des formes en M ou Y ainsi que des formes ayant une partie circulaire prolongée par une tige. Dans tous les modes de réalisation, la partie la plus large contient l'organe élastique et est destinée à être la plus interne ; lorsqu'elle se dilate, après sa sortie de l'applicateur, elle exerce une pression localisée au niveau du col vésical. Ces dispositifs ne sont pas destinés à pénétrer au-delà du col vésical.

Le document EP-1 279 381 décrit un dispositif de prévention de l'incontinence urinaire féminine à l'effort qui comprend une partie proximale et une partie distale qui la prolonge. Dans ce document, la partie distale est destinée à pénétrer dans la partie sous-urétrale du vagin et à s'élargir. Ce document ne donne aucune indication sur l'interaction de la vessie et de la partie distale. Dans ce dispositif, la partie proximale est destinée à se trouver dans la partie sous-urétrale du vagin et à exercer une pression sur l'urètre, entre le col vésical et le méat urinaire, de préférence sur une certaine longueur. La partie distale a pour rôle de positionner la partie proximale le long de la partie sous-urétrale du vagin afin qu'elle exerce une pression sur l'urètre.

Les dispositifs des deux premiers documents, ainsi que de nombreux autres, présentent l'inconvénient de soulever en permanence la jonction urétro-vésicale, alors que les fuites urinaires à l'effort ne surviennent qu'à de courts moments. Cette pression constante peut provoquer une déformation anatomique qui peut elle-même être la cause d'une gêne et même d'une douleur. En outre, le contact est extrêmement localisé sur une très petite zone de la paroi. De plus, dans le cas du premier document, un contact postérieur très localisé s'établit aussi et peut être lui-même la cause d'une gêne et même d'une douleur.

En outre, le positionnement, dans le cas des deux premiers documents, est très délicat ; si le dispositif est trop enfoncé, il est inefficace, et s'il n'est pas suffisamment enfoncé, il peut créer une gêne, voir une douleur, et peut même provoquer une impossibilité d'uriner.

En outre, dans le cas du premier document, le contact au niveau du col vésical s'effectue de part et d'autre de l'urètre et non directement contre celle-ci.

Le dispositif du dernier document précité EP-1 279 381 présente de nombreux avantages, notamment le fait d'être efficace grâce à son action sur une partie étendue de la longueur de l'urètre.

On a maintenant découvert un perfectionnement au dispositif du document EP-1 279 381, qui permet de tirer avantage dynamiquement des conditions propres à l'utilisatrice, et plus précisément du remplissage de la vessie pour adapter sa propre action.

On comprend facilement que les incidents d'incontinence urinaire sont plus fréquents lorsque la vessie est pleine que lorsqu'elle est vide. Selon l'invention, grâce à l'utilisation d'une partie distale de contour fermé, délimitant une surface étendue d'action du poids de la vessie, ce poids agit sur cette partie distale en fonction de l'état de remplissage de la vessie ; cette partie distale transmet alors, par un effet de levier, une force d'autant plus grande que la vessie est pleine, à la partie proximale qui agit en conséquence sur l'urètre.

Un avantage évident de cette caractéristique est que, puisque le dispositif s'adapte automatiquement à l'état de remplissage de la vessie, il ne crée qu'une gêne réduite, surtout lorsque la vessie est peu remplie.

Plus précisément, l'invention concerne un dispositif de prévention de l'incontinence urinaire féminine à l'effort destiné à être disposé de manière longitudinale dans le vagin, et qui comporte une partie proximale (12) s'étendant entre une première extrémité libre et une deuxième extrémité, réalisée de manière à être sensiblement indéformable sous pression et destinée à être placée dans la zone sous-urétrale du vagin sur une longueur notable de cette zone, et une partie distale (14) prolongeant la deuxième extrémité de la partie proximale (12), réalisée dans un matériau déformable sous pression de manière réversible, et destinée à être placée dans la zone sous-vésicale du vagin, la partie distale (14) délimitant un contour fermé. Un tel contour fermé délimite lui-même une surface étendue d'application du poids de la vessie.

De préférence, la partie proximale s'étend sur le tiers au moins de la longueur de la zone sous-urétrale, et, très avantageusement, sur les deux-tiers environ de la longueur de la zone sous-urétrale.

De préférence, le contour fermé de la partie distale a une forme d'anneau.

Il est avantageux que l'épaisseur de la partie distale soit plus faible à l'extrémité distale que du côté de la partie proximale.

De préférence, la partie distale est déformable élastiquement au moins entre une position de fonctionnement dans laquelle elle a une forme arrondie, et une position de mise en place dans laquelle ses parties latérales sont serrées à proximité l'une de l'autre dans le prolongement de la partie proximale.

De préférence, la partie proximale possède un noyau réalisé dans un matériau dur et entouré d'une enveloppe souple réalisée dans un matériau biocompatible. Le noyau a avantageusement une forme de tronçon de tube, et la partie proximale forme de préférence une cavité débouchant à la première extrémité.

La partie proximale présente de préférence une forme cylindrique ou tronconique.

De préférence, le dispositif a une longueur comprise entre 7 et 9 cm, et la partie distale a une largeur comprise entre 4 et 5,5 cm.

De préférence, le dispositif comporte en outre un dispositif pour son retrait, monté à la première extrémité de la partie proximale.

De préférence, la partie du dispositif formant la transition entre la partie proximale et la partie distale est déformable élastiquement.

L'invention concerne aussi un procédé de prévention de l'incontinence urinaire féminine à l'effort, qui comprend la transformation, par un effet de levier, du poids de la vessie en une force ascendante agissant sur l'urètre, par articulation à un emplacement de la cavité vaginale proche du col vésical.

D'autres caractéristiques et avantages de l'invention ressortiront mieux de la description qui va suivre d'exemples de réalisation, faite en référence aux dessins annexés sur desquels :
la figure 1 est une vue en perspective d'un dispositif de prévention de l'incontinence urinaire féminine à l'effort selon l'invention ;
la figure 2 est une vue en plan du dispositif de la figure 1 ;
la figure 3 est une vue de bout du dispositif de la figure 1 ;
la figure 4 est une coupe médiane du dispositif des figures 1 à 3, représentant une caractéristique avantageuse de l'invention ; et
la figure 5 est un schéma illustrant le fonctionnement du dispositif selon l'invention.

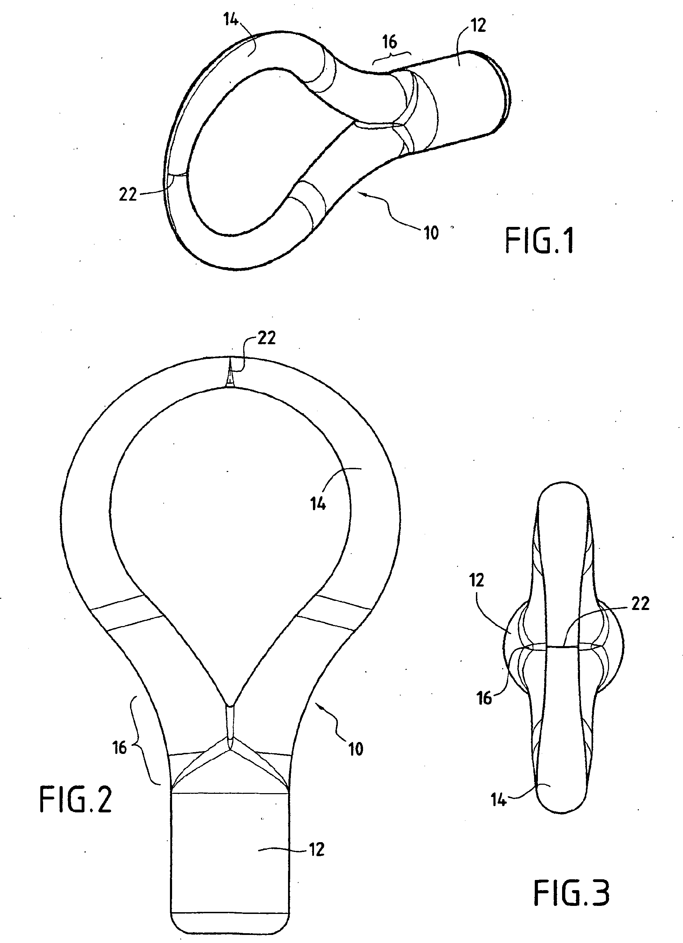

La figure 1 est une vue en perspective d'un dispositif de prévention de l'incontinence urinaire féminine à l'effort selon l'invention. Ce dispositif comporte une partie proximale 12 et une partie distale 14 en forme d'anneau pratiquement, avec une transition désignée par la référence 16. Le dispositif 10 comporte, dans sa partie proximale 12, un organe pratiquement rigide 18, avantageusement sous forme d'un tronçon de tube muni par exemple d'une traverse permettant l'accrochage d'un fil d'extraction.

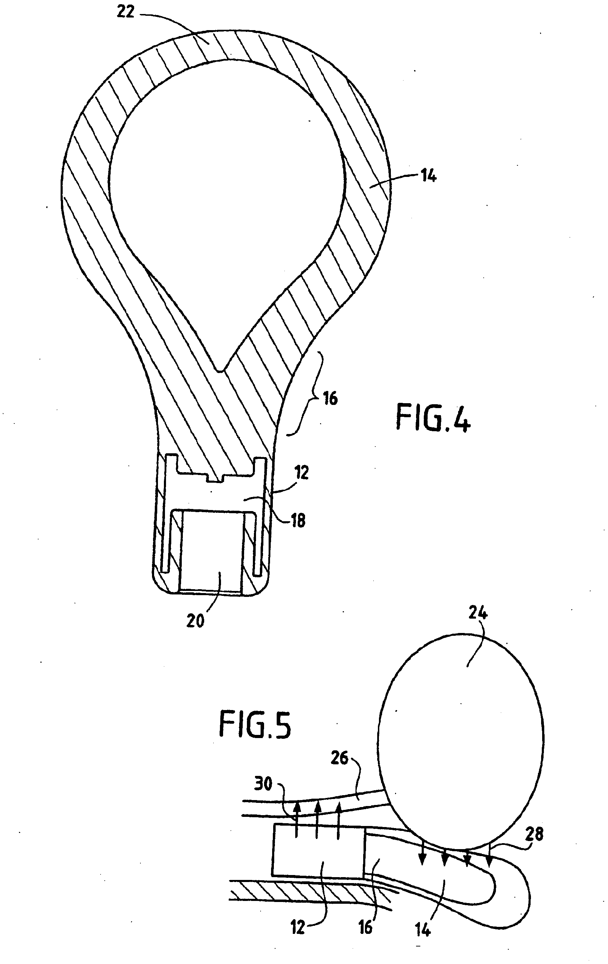

On note sur la figure 4 que l'organe rigide 18 est enrobé dans le matériau du dispositif 10, et il n'est exposé que par un orifice 20, de sorte qu'il n'est jamais au contact de tissus du corps dans lequel est placé le dispositif. A part l'organe rigide 18, la totalité du dispositif 10 est formée d'un matériau biocompatible flexible, par exemple du styrène-butadiène-éthylène-styrène (SBES).

La partie distale 14, qui donne avec la partie proximale une forme de raquette, a deux parties latérales qui se rencontrent à un sommet 22. Il est avantageux que l'épaisseur de la partie distale 14 diminue progressivement de la partie 16 de transition jusqu'à l'extrémité 22. De cette manière, la partie distale peut être facilement déformée, surtout au voisinage de l'extrémité 22 ; par exemple, les deux parties latérales de la partie distale 14 peuvent être rapprochées l'une de l'autre en donnant une forme effilée qui facilite l'insertion dans la partie sous-urétrale du vagin, même en l'absence d'un applicateur. En outre, cette insertion assure un positionnement convenable automatique du dispositif ; en effet, en s'écartant, les parties latérales tirent la partie proximale vers l'intérieur jusqu'à ce qu'elles soient en appui sur les parois latérales du vagin, car celui-ci s'élargit de l'extérieur vers l'intérieur.

Par ailleurs, le vagin étant plus large que haut dans sa partie profonde, le dispositif a tendance à se positionner de lui-même par rotation, même si les parties latérales de la partie distale 14 sont introduites avec une orientation verticale et non horizontale.

Il est cependant possible d'utiliser un applicateur dans lequel la partie distale 14 est serrée, l'ensemble du dispositif tenant dans un cylindre creux dont le diamètre interne est pratiquement égal au diamètre externe de la partie proximale 12.

La figure 5 indique comment agit le dispositif selon l'invention après qu'il a été mis en position. Sur cette figure, on a schématiquement représenté la vessie 24 et l'urètre 26. Lorsque la vessie se remplit, elle exerce une pression sur la paroi vaginale supérieure, comme indiqué par les flèches 28. Cette pression est appliquée de manière répartie sur la partie distale 14 qui tend à se déplacer vers le bas. Ce déplacement vers le bas de la partie distale 14 est transmis, par un effet de levier, à la partie proximale qui subit une force ascendante 30 vers l'urètre 26. En effet, la partie de transition 16 se trouve approximativement au niveau du col vésical, et elle est pratiquement en contact permanent avec les parois vaginales supérieure et inférieure ; elle constitue ainsi un point d'articulation qui permet la transformation de la force descendante 28 en une force ascendante 30. Grâce à l'élasticité du matériau flexible du dispositif 10, cette action s'effectue progressivement et sans douleur.

On conçoit facilement que, lorsque la vessie est vide ou presque vide, la force 28 est faible si bien que la partie proximale 12 exerce une force 30 réduite sur l'urètre 26. Cependant, comme la vessie est vide, il est très peu probable qu'un effort provoque une incontinence urinaire. En outre, en cas de rire ou de toux, la force abdominale s'exerce de manière sensiblement identique à la force 28 et provoque la projection de la partie proximale 12 contre l'urètre, pendant une fraction de seconde, et évite l'incontinence même dans ce cas.

Lorsque la vessie est pleine, tout son poids est transmis par la paroi vaginale supérieure à la partie distale 14 qui transmet élastiquement cette force à la partie proximale dont l'action sur l'urètre 26 est multipliée. En effet, on note que le bras de levier de la partie distale 14 est au moins le double de celui de la partie proximale 12. En conséquence, lorsque la vessie est pleine, l'effet de la partie proximale sur l'urètre 26 est plus que doublé.

Cette action intense se limite aux moments où la vessie est pleine. Lorsque celle-ci est pratiquement vide, la force exercée de manière permanente sur l'urètre 26 peut être très réduite et peut donc ne provoquer aucune gêne ni aucune douleur.

Dans un premier exemple de réalisation, la partie proximale 12 a une longueur d'environ 20 mm et le dispositif une longueur totale de 76 mm, la largeur de la partie distale étant d'environ 43 mm. Le diamètre de la partie proximale est de 15 mm.

Dans un second exemple, la partie proximale a une longueur d'environ 23 mm et le dispositif une longueur totale de 88 mm. La partie distale a une largeur d'environ 52 mm.

Dans ses deux exemples, le matériau flexible est du styrène-butadiène-éthylène-styrène (SBES), et le noyau rigide formé du tronçon de tube est du polyéthylène.

## Revendications

1. Dispositif de prévention de l'incontinence urinaire féminine à l'effort destiné à être disposé de manière longitudinale dans le vagin, comportant :
- une partie proximale (12) s'étendant entre une première extrémité libre et une deuxième extrémité, et
- une partie distale (14) prolongeant la deuxième extrémité de la partie proximale (12), réalisée dans un matériau déformable sous pression de manière réversible, et destinée à être placée dans la zone sous-vésicale du vagin, la partie distale (14) délimitant un contour fermé, **caracterisé en ce que** la partie proximal est réalisée de manière à être indéformable sous pression et destinée à être placée dans la zone sous-urétrale du vagin sur une longueur notable de cette zone de sorte que le
dispositif est apte à transformer, par un effet de levier, le poids de la vessie en une force ascendante agissant sur l'urètre, par articulation à un emplacement de la cavité vaginale proche du col vésical.

2. Dispositif selon la revendication 1, **caractérisé en ce que** la partie proximale s'étend sur le tiers au moins de la longueur de la zone sous-urétrale.

3. Dispositif selon la revendication 2, **caractérisé en ce que** la partie proximale s'étend sur les deux-tiers environ de la longueur de la zone sous-urétrale.

4. Dispositif selon l'une quelconque des revendications précédentes, **caractérisé en ce que** le contour fermé de la partie distale (14) a une forme d'anneau.

5. Dispositif selon la revendication 4, **caractérisé en ce que** l'épaisseur de la partie distale (14) est plus faible à l'extrémité distale que du côté de la partie proximale (12).

6. Dispositif selon l'une quelconque des revendications précédentes, **caractérisé en ce que** la partie distale (14) est déformable élastiquement au moins entre une position de fonctionnement dans laquelle elle a une forme arrondie, et une position de mise en place dans laquelle ses parties latérales sont serrées à proximité l'une de l'autre dans le prolongement de la partie proximale (12).

7. Dispositif selon l'une quelconque des revendications précédentes, **caractérisé en ce que** la partie proximale (12) possède un noyau (18) réalisé dans un matériau dur et entouré d'une enveloppe souple réalisée dans un matériau biocompatible.

8. Dispositif selon la revendication 5, **caractérisé en ce que** le noyau (18) a une forme de tronçon de tube, et la partie proximale (12) forme une cavité (20) débouchant à la première extrémité.

9. Dispositif selon l'une quelconque des revendications précédentes, **caractérisé en ce que** le dispositif (10) a une longueur comprise entre 7 et 9 cm, et la partie distale (14) a une largeur comprise entre 4 et 5,5 cm.

10. Dispositif selon l'une quelconque des revendications précédentes, **caractérisé en ce qu'**il comporte en outre un dispositif de retrait du dispositif monté à la première extrémité de la partie proximale (12).

11. Dispositif selon l'une quelconque des revendications précédentes, **caractérisé en ce que** la partie (16) du dispositif (10) formant la transition entre la partie proximale (12) et la partie distale (14) est déformable élastiquement.

12. Dispositif selon l'une quelconque des revendications précédentes, **caractérisé en ce que** la partie proximale (12) présente une forme choisie parmi les formes cylindrique et tronconique.

## Claims

1. Device for preventing enuresis during an effort in women, said device being intended to be longitudinally arranged inside vagina and comprising:
- a proximal part (12) extending between a first free end and a second end, and
- a distal part (14) in the extension of the second end of the proximal part (12), made out of a reversibly pressure-deformable material, and intended to be placed in the subvesical zone of vagina, the distal part (14) delimiting a closed contour,
**characterized in that** the proximal part is made so as to keep its shape under pressure and is intended to be placed in the suburethral zone of vagina over a good length of this zone so that the device is able to transform, according to a lever effect, the bladder weight into an upwards force articulatedly acting on the urethra at a place of the vaginal cavity closed to the vesical neck.

2. Device according to claim 1, **characterized in that** the proximal part extends over at least one third of the length of the suburethral zone.

3. Device according to claim 2, **characterized in that** the proximal part extends over about two thirds of the length of the suburethral zone.

4. Device according to anyone of the preceding claims, **characterized in that** the closed contour of the distal part (14) is ring-shaped.

5. Device according to claim 4, **characterized in that** the thickness of the distal part (14) is lower at the distal end than at the proximal part (12).

6. Device according to anyone of the preceding claims, **characterized in that** the distal part (14) is elastically deformable at least between a position of operation, in which it has a round shape, and a position of installation in which its lateral parts are tightened close to one another in the extension of the proximal part (12).

7. Device according to anyone of the preceding claims, **characterized in that** the proximal part (12) includes a core (18) made out of a hard material and surrounded by a flexible cover made out of a biocompatible material.

8. Device according to claim 5, **characterized in that** the core (18) has the shape of a tube section, and the proximal part (12) forms a cavity (20) opening at the first end.

9. Device according to anyone of the preceding claims, **characterized in that** the device (10) has a length between 7 and 9 cm, and the distal part (14) has a width between 4 and 5.5 cm.

10. Device according to anyone of the preceding claims, **characterized in that** it further comprises a device for removing the device mounted at the first end of the proximal part (12).

11. Device according to anyone of the preceding claims, **characterized in that** the part (16) of the device (10) forming the transition between the proximal part (12) and the distal part (14) is elastically deformable.

12. Device according to anyone of the preceding claims, **characterized in that** the proximal part (12) has a shape selected among a cylindrical shape and a truncated shape.

## Patentansprüche

1. Vorrichtung zum Vorbeugen der Harninkontinenz bei Anstrengung bei Frauen, wobei die genannte Vorrichtung longitudinal in der Vagina angeordnet werden soll, und umfasst:
- ein proximales Teil (12), das sich zwischen einem ersten freien Ende und einem zweiten Ende erstreckt, und
- ein distales Teil (14) in der Verlängerung des zweiten Endes des proximalen Teils (12), wobei es aus einem umkehrdruckverformbaren Material gebildet ist, und in die Subblasenzone der Vagina gelegt werden soll, wobei das distale Teil (14) einen geschlossenen Umriss abgrenzt,
**dadurch gekennzeichnet, dass** das proximale Teil derart gebildet ist, dass es druckformbeständig ist, und in die suburethrale Zone der Vagina über einer guten Länge dieser Zone angeordnet werden soll, sodass die Vorrichtung in der Lage ist, entsprechend einem Hebeleffekt das Blasengewicht in eine aufwärtsgerichtete, auf Urethra gelenkartig einwirkende Kraft an einem Ort des vaginalen Hohlraums in der Nähe der Blasenhals umzuwandeln.

2. Vorrichtung nach Anspruch 1, **dadurch gekennzeichnet, dass** sich das proximale Teil über mindestens ein Drittel der Länge der suburethralen Zone erstreckt.

3. Vorrichtung nach Anspruch 2, **dadurch gekennzeichnet, dass** sich das proximale Teil über etwa zweidrittel der Länge der suburethralen Zone erstreckt.

4. Vorrichtung nach einem der vorausgegangenen Ansprüche, **dadurch gekennzeichnet, dass** der geschlossene Umriss des distalen Teils (14) ringförmig ist.

5. Vorrichtung nach Anspruch 4, **dadurch gekennzeichnet, dass** die Stärke des distalen Teils (14) niedriger am distalen Ende als am proximalen Teil (12) ist.

6. Vorrichtung nach einem der vorausgegangenen Ansprüche, **dadurch gekennzeichnet, dass** das distale Teil (14) mindestens zwischen einer Betriebsstellung, in welcher es eine runde Form hat, und einer Installationsstellung, in welcher seine seitlichen Teile aneinander in der Verlängerung des proximalen Teils (12) geklemmt werden, elastisch nachgiebig ist.

7. Vorrichtung nach einem der vorausgegangenen Ansprüche, **dadurch gekennzeichnet, dass** das proximale Teil (12) einen durch eine flexible Abdeckung aus biokompatiblem Material umgebenen Kern (18) aus hartem Material umfasst.

8. Vorrichtung nach Anspruch 5, **dadurch gekennzeichnet, dass** der Kern (18) die Form eines Rohrabschnitts aufweist, und das proximale Teil (12) einen Hohlraum (20) bildet, der sich am ersten Ende öffnet.

9. Vorrichtung nach einem der vorausgegangenen Ansprüche, **dadurch gekennzeichnet, dass** die Vorrichtung (10) eine Länge zwischen 7 und 9 cm, und das distale Teil (14) eine Breite zwischen 4 und 5,5 cm aufweist.

10. Vorrichtung nach einem der vorausgegangenen Ansprüche, **dadurch gekennzeichnet, dass** sie weiter eine Vorrichtung für das Entfernen der am ersten Ende des proximalen Teils (12) angebauten Vorrichtung umfasst.

11. Vorrichtung nach einem der vorausgegangenen Ansprüche, **dadurch gekennzeichnet, dass** das Teil (16) der den Übergang zwischen dem proximalen Teil (12) und dem distalen Teil (14) bildenden Vorrichtung (10) elastisch nachgiebig ist.

12. Vorrichtung nach einem der vorausgegangenen Ansprüche, **dadurch gekennzeichnet, dass** das proximale Teil (12) eine Form aufweist, die unter einer zylinderförmigen Form und einer kegelstumpfartigen Form ausgewählt ist.
